# EUROPEAN PATENT APPLICATION

(11) **EP 4 643 825 A1**
(43) Date of publication of application: **05.11.2025**
(21) Application number: 23910397.1
(22) Date of filing: 22.12.2023
(51) Int. Cl.: A61F 2/90, A61F 2/856

(54) **LUMEN STENT**

(30) Priority: 29.12.2022 CN 202211709919
(71) Applicant: Lifetech Scientific (Shenzhen) Co., Ltd., Shenzhen, Guangdong 518063 (CN)
(72) Inventor: CHEN, Erchong, Shenzhen, Guangdong 518063 (CN)
(74) Representative: Michalski Hüttermann & Partner Patentanwälte mbB
(86) International application number: PCT/CN2023/141012
(87) International publication number: WO 2024/140471

(57) **Abstract**

Provided is a lumen stent, which includes a mesh cover. The mesh cover includes at least one column of hooking units. Each hooking unit includes a first hooking piece and a second hooking piece that are hooked to each other in sequence from a proximal end to a distal end. The first hooking piece includes one trough and two first wave rods connected to the trough, the second hooking piece includes one crest and two second wave rods connected to the crest, and the trough of the first hooking piece and the crest of the second hooking piece can move relative to each other in an axial direction. The mesh cover includes a proximal cover and a distal cover in the axial direction. In the proximal cover, a first wave rod that is close to a radial edge of the mesh cover in a hooking unit spans over an upper side of a second wave rod in the same hooking unit; and/or in the distal cover, a second wave rod that is close to the radial edge of the mesh cover in a hooking unit spans over an upper side of a first wave rod in the same hooking unit. In the present disclosure, mesh holes on radial sides of the proximal cover and/or the distal cover can be significantly deformed under an external force and expand sufficiently to facilitate passage of a guide wire and a bridging stent through the mesh holes.

## Description

### Technical Field

The present disclosure relates to the technical field of medical devices, and in particular, to a lumen stent.

### Background Art

Conventional open surgery for treating vascular diseases such as aortic aneurysm and aortic dissection has problems such as a large wound, high mortality, long operation duration, high incidence of postoperative complications and high surgical difficulty. Minimally invasive interventional surgery for treating vascular diseases has the advantages of small wound, high safety, and high efficacy. Therefore, it is recognized by doctors and patients and has become an important treatment method for vascular diseases. Interventional treatment refers to the implantation of a vascular stent into a diseased segment of a blood vessel of a patient by using a delivery system. The implanted vascular stent can expand to support a narrowed and occluded segment of a blood vessel or obstruct a rupture of a vascular dissection, reduce the elastic recoil and reshaping of the blood vessel, maintain unobstructed blood flow in a lumen, and avoid vascular stenosis.

When an aneurysm or arterial dissection is located near a branch vessel of the aorta, a stent with a groove may be implanted. The groove corresponds to the branch vessel, ensuring that blood in the aorta enters the branch vessel through the groove. This can not only support the narrowed and occluded segment of the blood vessel or obstruct the rupture of the vascular dissection, but also maintain unobstructed blood flow in a branch. For a blood vessel with a narrow or twisted true lumen, a mesh cover may further be correspondingly arranged on the groove. The mesh cover has a good supporting effect and can prevent the lumen from squeezing the groove to compress the operating space. However, the mesh cover is likely to obstruct a guide wire or a bridging stent.

### Summary of the Disclosure

In view of the deficiencies in the prior art, the present disclosure provides a lumen stent that can relieve the problem of obstruction to a guide wire or a bridging stent.

The present disclosure provides a lumen stent, which includes a mesh cover. The mesh cover includes at least one column of hooking units. Each hooking unit includes a first hooking piece and a second hooking piece that are hooked to each other in sequence from a proximal end to a distal end. The first hooking piece includes one trough and two first wave rods connected to the trough, the second hooking piece includes one crest and two second wave rods connected to the crest, and the trough of the first hooking piece and the crest of the second hooking piece are configured to move relative to each other in an axial direction. The mesh cover includes a proximal cover and a distal cover in the axial direction. In the proximal cover, a first wave rod closer to a radial edge of the mesh cover in a hooking unit spans over an upper side of a second wave rod in the same hooking unit; and/or in the distal cover, a second wave rod closer to the radial edge of the mesh cover in a hooking unit spans over an upper side of a first wave rod in the same hooking unit.

In an embodiment, a hooking gap is reserved between the first hooking piece and the second hooking piece in the same hooking unit.

In an embodiment, the trough and the crest include transverse rods, the two first wave rods are connected through the transverse rod, and/or the two second wave rods are connected through the transverse rod.

In an embodiment, the included angles between the first wave rods and the transverse rod are obtuse angles, and/or the included angles between the second wave rods and the transverse rod are obtuse angles.

In an embodiment, the transverse rod is a straight rod or an arc-shaped rod.

In an embodiment, the mesh cover includes a first mesh region and at least two second mesh regions respectively connected to two sides of the first mesh region in a circumferential direction of the lumen stent. Each second mesh region includes at least one column of hooking units, and the first mesh region includes a plurality of columns of cross units.

In an embodiment, an axial gap is reserved between the first wave rod and the second wave rod close to the radial edge of the mesh cover of the hooking unit. A plurality of axial gaps formed by the same column of hooking units include end gaps and a middle gap. The end gaps are closer to an axial end of the mesh cover compared with the middle gap. The maximum axial length of at least one end gap is greater than the maximum axial length of the middle gap.

In an embodiment, the mesh cover further includes at least one column of side connecting pieces arranged on a radial side of the mesh cover. The side connecting pieces are connected to the hooking units and include connecting holes for connection.

In an embodiment, the mesh cover further includes a side end connecting piece arranged at the axial end of the mesh cover. A plurality of side connecting pieces in a same column include two first side connecting pieces and a second side connecting piece between the two first side connecting pieces. Each first side connecting piece is connected to the side end connecting piece and the hooking unit, respectively, in the axial direction, and the second side connecting piece is connected to one first hooking piece and one second hooking piece, respectively.

In an embodiment, the lumen stent further includes a main stent. The main stent includes an inner cavity. Side surfaces of the main stent sink inward in a radial direction to form a groove. The mesh cover is connected to the groove, and a radial gap is reserved between at least part of the mesh cover and the bottom of the groove in the radial direction of the lumen stent and is communicated with the inner cavity.

In an embodiment, the mesh cover is connected to the main stent through the plurality of side connecting pieces, and a gap reserved between two adjacent side connecting pieces is communicated with the radial gap.

The present disclosure has the following beneficial effects. Compared with the prior art, the present disclosure provides the lumen stent, which includes the mesh cover. The mesh cover includes the at least one column of hooking units. Each hooking unit includes the first hooking piece and the second hooking piece that are hooked to each other in sequence from the proximal end to the distal end. The first hooking piece includes one trough and the two first wave rods connected to the trough, the second hooking piece includes one crest and the two second wave rods connected to the crest, and the trough of the first hooking piece and the crest of the second hooking piece are capable of moving relative to each other in the axial direction. The mesh cover includes the proximal cover and the distal cover in the axial direction. In the proximal cover, the first wave rod close to the radial edge of the mesh cover in the hooking unit spans over the upper side of the second wave rod in the same hooking unit; and/or in the distal cover, the second wave rod close to the radial edge of the mesh cover in the hooking unit spans over the upper side of the first wave rod in the same hooking unit. The wave rod at the top can be separated from the wave rod below in the radial direction of the lumen stent, and thus, the mesh cover has capability and space for upward deformation. In this way, mesh holes on the radial sides of the proximal cover and/or the distal cover can be significantly deformed under an external force and sufficiently expanded to facilitate passage of a guide wire and a bridging stent through the mesh holes. When the external force is withdrawn, the mesh holes can retract to support and limit the bridging stent, thereby reducing the possibility that the bridging stent swings with blood flow or heart beats and ensuring the stability of branch blood flow.

The present disclosure further provides a lumen stent, which includes a main stent and a mesh cover connected to the main stent. Side surfaces of the main stent sink inward in a radial direction to form a groove. A radial gap is reserved between at least part of the mesh cover and the bottom of the groove in the radial direction of the lumen stent. The mesh cover includes a side end connecting piece connected to the main stent and a middle side end connecting piece. The side end connecting piece is closer to a radial edge of the mesh cover compared with the middle end connecting piece. The middle end connecting piece at a proximal end of the mesh cover is closer to a proximal end of the lumen stent compared with the side end connecting piece at the proximal end of the mesh cover, and/or the middle end connecting piece at a distal end of the mesh cover is closer to a distal end of the lumen stent compared with the side end connecting piece at the distal end of the mesh cover.

In an embodiment, the middle end connecting piece is connected to an axial edge of the groove, and the side end connecting piece is connected to the axial edge and/or a radial edge of the groove.

In an embodiment, the middle end connecting piece includes a middle wave angle, which includes a vertex connected to an axial end of the groove and two wave rods extending from the vertex toward the other axial end of the groove.

In an embodiment, the main stent further includes a main membrane. A stent with two branch ports is arranged on an inner wall of the main stent. Branch ports of the stent with two branch ports are arranged close to one axial end of the groove, a gap position is reserved between the branch ports of the stent with two branch ports. The middle end connecting piece is connected to the main membrane at the gap position.

In an embodiment, a support piece is further arranged at a branch port edge of the stent with two branch ports, and at least a partial region of the support piece at the branch port edge is connected to the main membrane and is located on two sides of the middle end connecting piece.

In an embodiment, the mesh cover further includes a bending portion, which includes a first rod and a second rod that are connected to each other. A bending angle is formed between the first rod and the second rod. The first rod extends from the bending angle toward one axial end of the mesh cover, and the second rod extends from the bending angle toward the other axial end of the mesh cover.

In an embodiment, the middle end connecting piece includes a middle wave angle, which includes a vertex and a wave rod connected to the vertex. The wave rod spans over the upper side of the bending potion.

In an embodiment, the wave rod of the middle wave angle is in contact with the bending portion, or a gap is reserved between the wave rod of the middle wave angle and the bending portion in the radial direction of the lumen stent.

In an embodiment, the mesh cover includes a first mesh region and at least two second mesh regions respectively connected to two sides of the first mesh region. Each second mesh region includes at least one column of hooking units, and the first mesh region includes a plurality of columns of cross units.

In an embodiment, the vertex of the middle wave angle is opposite to the first mesh region in an axial direction, and two wave rods of the middle wave angle extend from the vertex of the middle wave angle in directions away from each other and are respectively connected to the second mesh regions on two sides of the first mesh region.

In an embodiment, an end that is away from the vertex of the middle wave angle, of the wave rod of the middle wave angle is connected to the hooking unit in the second mesh region.

In an embodiment, the side end connecting piece includes an edge wave angle, which includes a vertex. The vertex of the edge wave angle is connected to a corner of the groove.

The present disclosure has the following beneficial effects. Compared with the prior art, because the middle end connecting piece is connected to the main stent, when bent, the main stent can effectively drive the mesh cover to bend and deform to conform with the shape of a blood vessel. Furthermore, the middle end connecting piece connected to the main stent can provide a good support force for the axial end of the groove, thereby avoiding the formation of a gap between the main stent and the middle end connecting piece in the axial direction when the lumen stent is bent. In this way, the possibility that an inner wall of a blood vessel with a narrow true lumen squeezes the gap and enters the groove through the gap, which may prevent a guide wire and a bridging stent from entering a branch stent, is avoided.

The present disclosure further provides a lumen stent, which includes a main stent and a mesh cover connected to the main stent. Side surfaces of the main stent sink inward in a radial direction to form a groove. A radial gap is reserved between at least part of the mesh cover and the bottom of the groove in the radial direction of the lumen stent. The mesh cover includes a bending portion, which includes a first rod and a second rod that are connected to each other. A bending angle is formed between the first rod and the second rod. The first rod extends from the bending angle toward one axial end of the mesh cover, and the second rod extends from the bending angle toward the other axial end of the mesh cover.

In an embodiment, the bending angle is an obtuse angle and is bent toward the axial end close to the bending portion of the mesh cover.

In an embodiment, the first rod extends toward one radial edge of the mesh cover, and the second rod extends toward the other radial edge of the mesh cover.

In an embodiment, an end that is away from the bending angle of the first rod is connected to an edge of the groove.

In an embodiment, the axial end of the mesh cover further includes an edge wave angle, which includes a vertex and two wave rods connected to the vertex. The first rod serves as one wave rod, and the other wave rod extends from the vertex toward the axial end away from the vertex of the mesh cover. An included angle is formed between the two wave rods at the vertex.

In an embodiment, the edge wave angle is connected to a corner of the groove.

In an embodiment, the mesh cover includes a first mesh region and at least two second mesh regions respectively connected to two sides of the first mesh region. Each second mesh region includes at least one column of hooking units, and the first mesh region includes a plurality of columns of cross units.

In an embodiment, the second rod passes through the first mesh region and is connected to the hooking unit in the second mesh regions.

In an embodiment, the axial end of the mesh cover includes two edge wave angles and a middle wave angle between the two edge wave angles. The middle wave angle is connected to the axial end of the groove.

In an embodiment, the middle wave angle spans over the bending portion.

The present disclosure has the following beneficial effect. Because the first rod and the second rod of the bending portion respectively extend toward the opposite axial ends of the mesh cover, compared with a straight rod, when the mesh cover is squeezed by a wall of a blood vessel in a radial direction and is bent to conform with a shape of the blood vessel (such as a shape of a greater curvature of the aortic arch), the first rod and the second rod that form the bending angle can move relative to each other, so that the bending portion can bulge away from the groove. The mesh cover is more likely to form an arch in this region to avoid compression of the space in the groove, so that a guide wire and a bridging stent can easily enter the groove.

### Brief Description of the Drawings

FIG. 1 is a schematic diagram of a lumen stent of the present disclosure;
FIG. 2 is a schematic structural diagram of an unfolded mesh cover of the present disclosure;
FIG. 3 is a schematic structural diagram of an unfolded mesh cover according to another embodiment of the present disclosure;
FIG. 4 is a stereoscopic structural diagram of a mesh cover of the present disclosure;
FIG. 5 is a partial enlarged view of position A in FIG. 4 of the present disclosure;
FIG. 6 is a partial enlarged view of position B in FIG. 4 of the present disclosure;
FIG. 7 is a partial enlarged view of position C in FIG. 4 of the present disclosure;
FIG. 8 is a partial enlarged view of position D in FIG. 4 of the present disclosure;
FIG. 9 is a stereoscopic structural diagram of a mesh cover according to another embodiment of the present disclosure;
FIG. 10 is a partial enlarged view of position E in FIG. 9 of the present disclosure;
FIG. 11 is a stereoscopic structural diagram of a mesh cover according to still another embodiment of the present disclosure;
FIG. 12 is a schematic stereoscopic diagram of a proximal section of a lumen stent of the present disclosure;
FIG. 13 is a partial enlarged view of position F in FIG. 12 of the present disclosure;
FIG. 14 is a schematic diagram of suturing of a middle wave angle and a proximal main membrane according to an embodiment of the present disclosure; and
FIG. 15 is a partial enlarged view of position G in FIG. 14 of the present disclosure.

### Detailed Description of the Disclosure

To better understand the concept of the present disclosure, implementations of the present disclosure are specifically described below with reference to the drawings. The following specific embodiments are merely some embodiments of the present disclosure, and are not intended to limit the present disclosure.

For ease of description, spatial relative relationship terms may be used herein to describe a relationship of one element or feature relative to another element or feature as shown in a figure. These relative relationship terms include, for example, "interior", "exterior", "inside", "outside", "under", "below", "on", and "upper side". Such spatial relative relationship terms are intended to encompass different orientations of an apparatus in use or operation in addition to an orientation depicted in the figure. For example, if the apparatus in the figure is turned over, an element described as "under" or "below" another element or feature would then be oriented "on" or "above" the another element or feature. Thus, the exemplar term "below" can encompass both an orientation of above and below. The apparatus may be oriented in another way (rotated 90 degrees or at another orientation) and is accordingly interpreted by using a spatial relative relationship descriptor herein.

To more clearly describe a structure of the present application, the terms "proximal" and "distal" are defined herein as commonly used terms in the field of interventional medicine. Specifically, a "distal end" refers to an end where blood flows out, and a "proximal end" refers to an end where blood flows in. For example, after a stent is implanted, blood flows from a proximal end of the stent to a distal end of the stent. An "axial direction" refers to a length direction of the stent, and a "radial direction" refers to a direction perpendicular to the "axial direction". A radial direction of a mesh cover or a groove refers to a width direction of the mesh cover or the groove.

In the present disclosure, a "wave ring" (also called a waveform loop) is a closed ring structure, and a "waveform unit" is an arc-shaped structure. The "wave ring", the "waveform unit", and the "mesh cover" are prepared by weaving or cutting a metal elastic material or a polymer material. The metal elastic material includes materials known in the field of implantable medical instruments or a combination of various biocompatible materials, such as an alloy of two or more of cobalt, chromium, nickel, titanium, magnesium, and iron, 316L stainless steel, a tantalum-nickel-tantalum alloy, or other biocompatible metal elastic materials. The polymer materials include biocompatible materials such as polylactic acid. The "wave ring", the "waveform unit", and the "mesh cover" all have radial expansion capabilities, can contract in a radial direction under an external force, and can self-expand or can be mechanically expanded (for example, by balloon expansion) to restore to an original shape and maintain the original shape after the external force is withdrawn. In this way, after being implanted into a lumen, a stent is adhered to an inner wall of the lumen by virtue of a radial support force of the wave ring or the waveform unit or the mesh cover. A shape of a wave in the "wave ring" or the "waveform unit" is not limited, and the wave may be a Z-shaped wave, an M-shaped wave, a V-shaped wave, a sine wave, or the like. The "wave ring" and the "waveform unit" both include a plurality of crests (also called proximal vertices), a plurality of troughs (also called distal vertices), and wave rods for connecting the crests to the adjacent troughs. One vertex (the proximal vertex or the distal vertex) and two wave rods connected to the vertex form one wave.

In the present disclosure, a "membrane" can isolate liquid to a certain extent, which may be made from a biocompatible polymer material such as polytetrafluoroethylene (PTFE) or polyethylene terephthalate (PET).

### Embodiment I

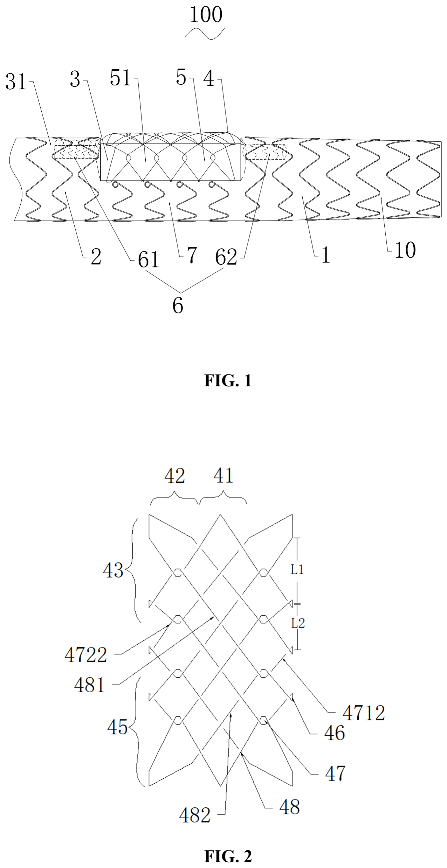

Referring to FIG. 1, in this embodiment, a lumen stent 100 includes a main stent 10 and a mesh cover 4 connected to the main stent 10. The entire main stent 10 is of a hollow tubular structure with ports at two ends, and side surfaces of the main stent sink inward in a radial direction to form a groove 5. The mesh cover 4 is connected to the groove 5, and a radial gap is reserved between at least part of the mesh cover 4 and the bottom 51 of the groove 5 in the radial direction of the lumen stent 100 and is communicated with an inner cavity of the main stent 10. The lumen stent 100 of the present disclosure is adaptive to a variety of blood vessels. In the embodiments of the present disclosure, the description is made by using an example in which the aortic arch is a target lumen for implantation. The aortic arch has a greater curvature (a side with a lower degree of bending and a larger curvature radius) and a lesser curvature (a side with a higher degree of bending and a smaller curvature radius). When the lumen stent 100 is implanted in the aortic arch, the mesh cover 4 faces the greater curvature and is opposite to a branch blood vessel, and an external bridging stent is connected to the branch blood vessel, passes through mesh holes of the mesh cover 4, and is communicated with the main stent 10 through the groove 5.

The main stent 10 includes a main support portion and a main membrane 31. The main membrane 31 may be arranged on an inner surface and/or an outer surface of the main support portion, or the main membrane 31 may be arranged on a part of the inner surface and a part of the outer surface of the main support portion.

Referring to FIG. 1, the main stent 10 can be divided into a proximal section 2, a distal section 1, and a middle section 7 between the proximal section 2 and the distal section 1. The proximal section 2 includes a tubular proximal support portion and a proximal main membrane, and the proximal main membrane may be covered on an inner side and/or an outer side of the proximal support portion by suturing, bonding, hot melting, or another method. The proximal support portion includes a plurality of main wave rings arranged at intervals in an axial direction. The distal section 1 includes a tubular distal support portion and a distal main membrane, and the distal main membrane may be covered on an inner side and/or an outer side of the distal support portion by suturing, bonding, hot melting, and another method. The distal support portion includes a plurality of main wave rings arranged at intervals in the axial direction.

The middle section 7 includes a middle unit and at least one mesh cover 4. The middle unit includes a middle main membrane 3 and a middle support portion (which may be omitted in other embodiments). An inner cavity enclosed by the middle main membrane 3 is communicated with an inner cavity enclosed by the proximal main membrane and an inner cavity enclosed by the distal main membrane. Side surfaces of the middle unit sink inward in a radial direction to form the groove 5. A part of the middle main membrane 3 serves as a membrane at the bottom 51 of the groove 5. A bottom support piece may further be arranged at the bottom 51 of the groove 5 and may include one or more of a waveform unit and a mesh structure. In other embodiments, the bottom support piece may be omitted. Two radial sides of the mesh cover 4 are fixedly connected to the middle main membrane 3 by suturing, bonding, hot melting, or another method. A radial gap (also called space, clearance, or cavity) is reserved between at least part of the mesh cover 4 and an outer surface of the middle unit 51.

A branch stent 6 may be arranged in the proximal section 2 and/or the distal section 1. An inner cavity of the branch stent 6 is communicated with the inner cavity of the main stent 10, and is communicated with the radial gap reserved between the bottom 51 of the groove 5 and the mesh cover 4. A plurality of branch stents 6 may be arranged. For example, branch stents 6 may be respectively arranged close to a proximal end of the groove 5 and close to a distal end of the groove 5.

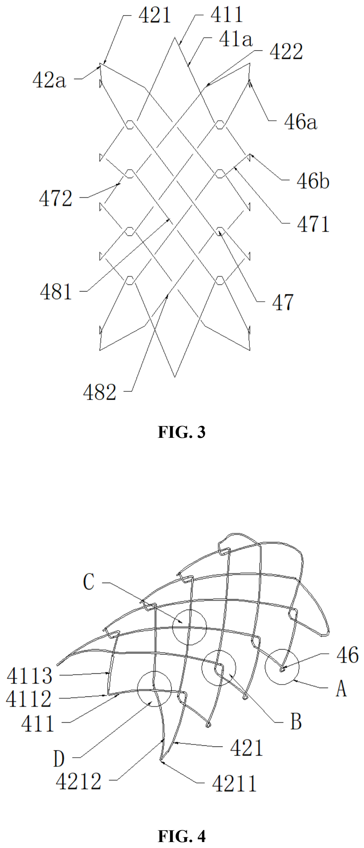

Referring to FIG. 2 and FIG. 4, in an embodiment, the mesh cover 4 is of an arc-shaped structure in a circumferential direction of the lumen stent 100, which is a mesh structure prepared by weaving of weaving wires. For example, the mesh cover 4 may be prepared by integrated weaving of weaving wires, or by separate weaving and splicing. In other embodiments, the mesh cover 4 may further be prepared by cutting. The central angle corresponding to a projection of the mesh cover 4 onto a radial plane of the lumen stent 100 may be less than or equal to 180 degrees, so that the mesh cover 4 has a good radial support force, and sufficient space is reserved in the middle unit for blood flow.

In this embodiment, the projection of an edge of the groove 5 onto a plane passing through two radial edges of the groove is roughly rectangular (which may also be understood that an opening of the groove 5 is roughly rectangular). The groove 5 includes a first edge, a second edge, a third edge, and the fourth edge. The opening of the groove 5 is enclosed by the four edges. The first edge is arranged opposite to the second edge in the radial direction of the lumen stent 100, extension directions of the first edge and the second edge are identical to a length direction of the lumen stent 100, and the third edge is arranged opposite to the fourth edge in the axial direction of the lumen stent 100. It may be understood that in other embodiments, the opening of the groove 5 may be in any other appropriate shape. For example, the first edge and the second edge may be at a certain angle to the axis of the lumen stent 100, so that the opening of the groove 5 is roughly trapezoidal, or the first edge and the second edge may be arcs, so that the opening of the groove 5 is roughly oval. The specific shape of the groove 5 is not limited in the present disclosure. Referring to FIG. 2 and FIG. 3, in this embodiment, the mesh cover 4 includes a first mesh region 41 and a second mesh region 42 that are connected to each other in a circumferential direction of the lumen stent 100. The first mesh region 41 and the second mesh region 42 both can contract in the radial direction under an external force, and can self-expand or can be mechanically expanded (for example, by balloon expansion) to restore to an original shape and maintain the original shape after the external force is withdrawn. Preferably, the mesh cover 4 includes the first mesh region 41 and two second mesh regions 42 respectively connected to two sides of the first mesh region 41 in the circumferential direction of the lumen stent 100.

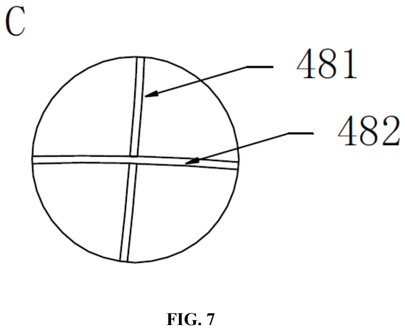

Referring to FIG. 7, the first mesh region 41 includes a plurality of first directional support wires 481 arranged at intervals and a plurality of second directional support wires 482 arranged at intervals. The first directional support wires 481 extend substantially in a first direction, and the second directional support wires 482 extend substantially in a second direction. The first directional support wires 481 are overlapped (or woven) with the second directional support wires 482 to form a plurality of columns of mesh holes and a plurality of columns of cross units 48. Each column of mesh holes includes a plurality of mesh holes substantially arranged in the axial direction. Each column of cross units 48 include a plurality of cross units 48 substantially arranged at intervals in the axial direction. The mesh hole is roughly rhombic, or may be in another shape such as a square or a rectangle. Four cross units 48 are correspondingly arranged at four corners of the mesh hole. Each cross unit 48 includes a cross point formed by mutual overlapping of the first directional support wire 481 and the second directional support wire 482. The first directional support wire 481 and the second directional support wire 482 can move relative to each other at this cross point. In some cross units 48, the first directional support wires 481 are located outside the second directional support wires 482, and in some cross units 48, the first directional support wires 481 are located inside the second directional support wires 482. In other embodiments, in the first mesh region 41, the first directional support wires 481 are all located outside the second directional support wires 482, or the first directional support wires 481 are all located inside the second directional support wires 482. Because the first directional support wires 481 and the second directional support wires 482 are overlapped with each other and can move relative to each other at the cross points of the cross units 48, the mesh holes in the first mesh region 41 are deformed under an external force and expanded to facilitate passage of a guide wire and a bridging stent through the mesh holes. After the force is withdrawn, the mesh holes can retract to support and limit the bridging stent, thereby reducing the possibility that the bridging stent swings with blood flow or heart beats and ensuring the stability of branch blood flow.

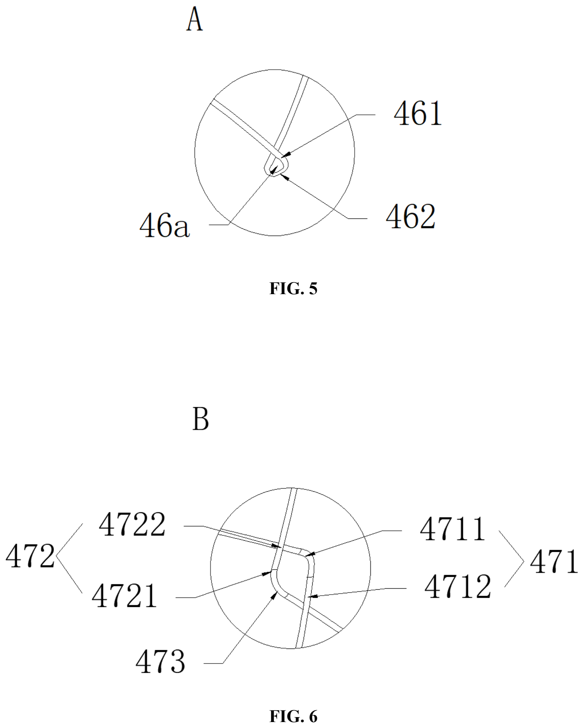

Referring to FIG. 6. Each second mesh region 42 includes at least one column of hooking units 47. Each column of hooking units 47 include a plurality of hooking units 47 arranged in sequence in the axial direction. Each hooking unit 47 includes a first hooking piece 471 and a second hooking piece 472. The first hooking piece 471 and the second hooking piece 472 are a part of the support wire. The first hooking piece 471 includes one wave bulging toward a distal end, that is, includes one trough 4711 (also called a distal vertex) and two first wave rods 4712 connected to the trough 4711; and the second hooking piece 472 includes one wave bulging toward a proximal end, that is, includes one crest 4721 (also called a proximal vertex) and two second wave rods 4722 connected to the crest 4721. In this embodiment, the first hooking piece 471 and the second hooking piece 472 in each hooking unit 47 are substantially hooked to each other in the axial direction. It should be noted that "substantially in the axial direction" herein refers to a connecting line between the distal vertex of the first hooking piece 471 and the proximal vertex of the second hooking piece 472 that is parallel to the axis of the mesh cover 4, or an included angle between the connecting line and the axis of the mesh cover 4 that is less than or equal to 45°.

A mutual hooking state of the first hooking piece 471 and the second hooking piece 472 at least includes a first hooking state and a second hooking state. The first hooking state refers to the following: in a natural unfolded state, the first hooking piece 471 is hooked to the second hooking piece 472, and a hooking gap L0 is reserved between the trough 4711 of the first hooking piece 471 and the crest 4721 of the second hooking piece 472 (that is, spaced at a distance). In this case, the first hooking piece 471 can move toward the proximal end or the distal end, and the second hooking piece 472 can also move toward the proximal end or the distal end. However, the hooking gap L0 limits the distance that the first hooking piece 471 moves toward the proximal end and the distance that the second hooking piece 472 moves toward the distal end. It may be understood that the hooking gap can further form a mesh hole structure for the bridging stent to enter when necessary. The second hooking state refers to the following: in the natural unfolded state, the first hooking piece 471 is hooked to the second hooking piece 472, and the trough 4711 of the first hooking piece 471 abuts against the crest 4721 of the second hooking piece 472 (that is, the hooking gap L0 is equal to 0). In this case, the movement of the first hooking piece 471 toward the proximal end and the movement of the second hooking piece 472 toward the distal end are constrained.

Referring to FIG. 2, the mesh cover 4 includes a proximal cover 43 and a distal cover 45 in the axial direction from the proximal end to the distal end. Referring to FIG. 6, FIG. 9, and FIG. 11, in the case that the first hooking piece 471 is hooked to the second hooking piece 472 to form the hooking unit 47, the hooking unit 47 in the proximal cover 43 is different from the hooking unit 47 in the distal cover 45. In the proximal cover 43, a first wave rod 4712 that is closer to a radial edge of the mesh cover 4 in a hooking unit 47 spans over an upper side of a second wave rod 4722 in the same hooking unit 47; and/or in the distal cover 45, a second wave rod 4722 that is closer to the radial edge of the mesh cover 4 in a hooking unit 47 spans over an upper side of a first wave rod 4712 in the same hooking unit 47. Referring to FIG. 2, in an unfolded structure, the upper side refers to the side away from the inner cavity of the main stent 10, which may also be understood as the outer side of the arch structure of the mesh cover 4. The reason for such an arrangement is that the bridging stent usually enters the groove 5 through the mesh holes on the proximal cover 43 and then is connected to the branch stent 6 at the proximal section 2. Because in the proximal cover 43, the first wave rod 4712 that is closer to the radial edge of the mesh cover 4 in the hooking unit 47 spans over the upper side of the second wave rod 4722 in the same hooking unit 47, the first wave rod 4712 can be separated from the second wave rod 4722 below in the radial direction of the lumen stent 100. Therefore, the first wave rod 4712 has capability and space for upward deformation. In this way, the mesh holes on the radial sides of the proximal cover 43 can be significantly deformed under an external force and sufficiently expanded to facilitate passage of the guide wire and the bridging stent through the mesh holes. After the external force is withdrawn, the mesh holes can retract to support and limit the bridging stent, thereby reducing the possibility that the bridging stent swings with blood flow or heart beats and ensuring the stability of branch blood flow. On the contrary, if the second wave rod 4722 at this position spans over the upper side of the first wave rod 4712, the first wave rod 4712 is restricted by the second wave rod 4722 above, thereby making it difficult for the first wave rod to be further lifted. In this way, the first hooking piece 471 and the second hooking piece 472 are twisted together at the hooking position, which is not conducive to the expansion and deformation of the mesh holes, thereby affecting entry of the bridging stent.

In the distal cover 45, the second wave rod 4722 that is close to the radial edge of the mesh cover 4 in the hooking unit 47 spans over the upper side of the first wave rod 4712 in the same hooking unit 47. The application principle and effectiveness of this structure are similar to those of the proximal cover 43, and details are not repeated herein again.

It may be understood that, to allow the mesh holes on the radial sides of the mesh cover 4 to be expanded and deformed easily, it is not required that the structure of the first mesh region 41 be the same as that described in this embodiment. In other embodiments, the structure of the first mesh region 41 may be similar to that of the second mesh region 42 of this embodiment, and include at least one column of hooking units 47, or the structure of the first mesh region 41 may be any appropriate structure.

Referring to FIG. 2 and FIG. 4, in an embodiment, an axial gap is reserved between the first wave rod 4712 and the second wave rod 4722 close to the radial edge of the mesh cover 4 of the hooking unit 47. Maximum axial lengths of axial gaps formed by a same column of hooking units 47 may be equal or different. Exemplarily, in this embodiment, the maximum axial lengths of the axial gaps formed by the same column of hooking units 47 are different. For example, the plurality of axial gaps formed by the plurality of hooking units 47 include end gaps and middle gaps. The end gaps are closer to the axial ends of the mesh cover 4 compared with the middle gaps. A maximum axial length (L1 shown in FIG. 2) of at least one end gap is greater than a maximum axial length (L2 shown in FIG. 2) of the middle gap. For example, the maximum axial length of the end gap close to the proximal end of the mesh cover 4 is greater than the maximum axial length of the middle gap, and the maximum axial length of the end gap close to the distal end of the mesh cover 4 is greater than the maximum axial length of the middle gap. In other embodiments, if the branch stent 6 is arranged close to only one axial end of the mesh cover 4, the maximum axial length of the end gap close to that axial end is greater than the maximum axial length of the middle gap. The axial end of the mesh cover 4 is opposite to the edge of the branch stent 6, so that there is a high probability that the bridging stent enters the branch stent 6 through the mesh hole close to the axial end of the mesh cover 4. By arranging the maximum axial length L1 of the end gap to be greater than the maximum axial length L2 of the middle gap, the size of the mesh hole close to the axial end of the mesh cover 4 is increased to facilitate entry of the guide wire and the bridging stent. It may be understood that to allow the mesh holes on the radial sides of the mesh cover 4 to be expanded and deformed easily, it is not required that the maximum axial length L1 of the end gap be greater than the maximum axial length L2 of the middle gap. The maximum axial length L1 of the end gap may be basically equal to the maximum axial length L2 of the middle gap. As long as in the proximal cover 43, the first wave rod 4712 that is closer to the radial edge of the mesh cover 4 in the hooking unit 47 spans over the upper side of the second wave rod 4722 in the same hooking unit 47; and/or in the distal cover 45, the second wave rod 4722 closer to the radial edge of the mesh cover 4 in the hooking unit 47 spans over the upper side of the first wave rod 4712 in the same hooking unit 47, the effect of easily deforming and expanding the mesh holes on the radial side of the mesh cover 4 can be achieved.

Further, in this embodiment, the mesh cover 4 further includes at least one column of side connecting pieces 46 arranged on the radial side of the mesh cover 4. Each column of side connecting pieces 46 includes at least one side connecting piece 46. The mesh cover 4 is connected to the main stent 10 through the side connecting piece 46. Exemplarily, the side connecting piece 46 includes a connecting hole 46a. The mesh cover 4 may be connected to the main stent 10 by suturing and through the connecting hole 46a. In other embodiments, the mesh cover may be fixedly connected to the main stent by bonding or another method.

Referring to FIG. 2, the side connecting pieces 46 are all connected to the hooking units 47. For example, the side connecting pieces 46 are respectively connected to two adjacent hooking units 47 in the axial direction. In the adjacent hooking units 47, the second wave rod 4722 of the second hooking piece 472 of the hooking unit 47 closer to the proximal end of the lumen stent 100 continues to extend and is bent to form the connecting hole, and then is connected to the first wave rod 4712 of the first hooking piece 471 of the hooking unit 47 closer to the distal end of the lumen stent 100. The first wave rod 4712 and the second waver rod 4722 in the hooking units 47 are both wave rods closer to the radial side of the mesh cover 4. The connecting hole is configured to fixedly connect the mesh cover to the membrane or the main stent by suturing. The connecting hole is not only conducive to suturing, but also achieves a limiting effect to prevent a suture and the mesh cover 4 from sliding relative to each other. In this embodiment, the connecting hole is a closed hole, which further improves the limiting effect. In other embodiments, the connecting hole may be an open hole.

Referring to FIG. 4 and FIG. 5, exemplarily, in this embodiment, the side connecting piece 46 is roughly triangular, and includes two waists 461 and a bottom side 462 connecting the two waists 461. The bottom side 462 substantially extends in the axial direction and is connected to the radial edge of the groove 5. One first wave rod 4712 of the first hooking piece 471 extends to form one waist 461, and one second wave rod 4722 of the second hooking piece 472 that is adjacent to, but is not hooked to the first hooking piece 471, extends to form the other waist 461. The two waists 461 of the side connecting piece 46 intersect to form a vertex. The two waists 461 are overlapped with and fixed to each other at the position of the vertex, for example, are fixed by suturing using a suture. By this connection method, the two radial sides of the mesh cover 4 have good stability, so as to ensure the good supporting capacity of the entire mesh cover 4 after the mesh cover is connected to the main stent. In another embodiment, if the side connecting pieces 46 are connected to two radial edges of the groove 5 by suturing or bonding, the bottom side 462 is connected to the edge of the groove 5, the two waists 461 can slide relative to each other at the intersection, which helps increase an expansion size of the mesh hole on the side of the mesh cover 4.

In other embodiments, the side connecting piece 46 may be in another shape. For example, the side connecting piece 46 includes one wave bulging toward the radial side of the mesh cover 4, the wave shares one wave rod with the first hooking piece 471, and shares one wave rod with the second hooking piece 472. One mesh hole is enclosed by one side connecting piece 46 and the first hooking piece 471 and the second hooking piece 472 that are connected to the side connecting piece 46. The side connecting piece 46 may be fixedly connected to the main stent 10 by suturing, bonding, and another method. Because the side connecting piece 46 has the vertex protruding toward the radial side of the mesh cover 4, and the side connecting piece 46 is fixedly connected to the edge of the groove 5 at the vertex, so the connection is secure. In other embodiments, the side connecting piece 46 may be in any appropriate shape such as a drop shape, a circle, an oval, or any other suitable shapes.

Referring to FIG. 2, in this embodiment, the mesh cover 4 further includes a side end connecting piece 42a arranged at the axial end of the mesh cover 4. For example, the side connecting pieces 42a are arranged at both the distal end and the proximal end of the mesh cover 4. The side end connecting piece 42a is connected to the edge of the groove 5. The foregoing side connecting piece 46 is arranged between the side end connecting piece 42a at the distal end of the mesh cover 4 and the side end connecting piece 42a at the proximal end of the mesh cover 4, and the side end connecting pieces 42a and the side connecting piece 46 are arranged at intervals. By arranging the side end connecting piece 42a, the mesh cover 4 can be bent and deformed along with the main stent 10, to effectively fit a blood vessel and provide support for the groove 5. In addition, in this embodiment, the side end connecting piece 42a includes an edge wave angle 421 bulging toward the axial end of the mesh cover 4. The mesh cover is fixedly connected to the main stent 10 through the edge wave angle 421, for example, by suturing, bonding, or another method. A closed connecting hole structure is not formed at a vertex 4211 of the edge wave angle 421, so that the sheath size at corners of the mesh cover 4 can be reduced, but also a large polygonal mesh hole can be formed by the edge wave angle 421 and the adjacent hooking unit 47. The large polygonal mesh hole is conducive to entry of the guide wire and the bridging stent through the mesh hole. In other embodiments, the side end connecting piece 42a may be omitted.

If the maximum axial length of at least one end gap is greater than the maximum axial length of the middle gap, because the side end connecting piece 42a is connected to the hooking unit 47, the maximum gap distance between the side end connecting piece 42a and the adjacent side connecting piece 46 is also greater than the maximum gap distance between two adjacent side connecting pieces 46, which helps increase the size of the side mesh hole close to the axial end of the mesh cover 4 to facilitate entry of the guide wire and the bridging stent. In other embodiments, the maximum gap distance between the side end connecting piece 42a and the adjacent side connecting piece 46 may be basically equal to the maximum gap distance between two adjacent side connecting pieces 46.

### Embodiment II

In this embodiment, the structure of a lumen stent 100 is basically the same as that of the lumen stent in Embodiment I. The difference is that a side end connecting piece 42a is connected to a side connecting piece 46.

Referring to FIG. 3, a plurality of side connecting pieces 46 include two first side connecting pieces 46a and a second side connecting piece 46b between the two first side connecting pieces 46a. The second side connecting piece 46b is connected to one first hooking piece 471 and one second hooking piece 472, respectively. Each first side connecting piece 46a is connected to the side end connecting piece 42a and a hooking unit 47, respectively, in an axial direction. Exemplarily, the first side connecting piece 46a close to a proximal end of the mesh cover 4 is connected to the side end connecting piece 42a and the first hooking piece 471 that are located at the proximal end of the mesh cover 4 respectively in the axial direction. For example, in the first hooking piece 471 of the hooking unit 47 at the most proximal end, a first wave rod 4712 closer to a radial edge of the mesh cover 4 continues to extend toward the proximal end of the mesh cover 4 and is bent in an opposite direction to form the first side connecting piece 46a, and then is connected to the side end connecting piece 42a at the proximal end of the mesh cover 4. The first side connecting piece 46a close to the distal end of the mesh cover 4 is connected to the side end connecting piece 42a and the second hooking piece 472 that are located at the distal end of the mesh cover 4 respectively in the axial direction. For example, in the second hooking piece 472 of the hooking unit 47 at the most distal end, a second wave rod 4722 closer to the radial edge of the mesh cover 4 continues to extend toward the distal end of the mesh cover 4 and is bent in an opposite direction to form the first side connecting piece 46a, and then is connected to the side end connecting piece 42a at the distal end of the mesh cover 4. In this embodiment, the side end connecting piece 42a includes an edge wave angle 421 bulging toward an axial end of the mesh cover 4. A large polygonal mesh hole formed by the edge wave angle 421 and the adjacent hooking unit 47 is conducive to entry of a guide wire and a bridging stent through the mesh hole. In this embodiment, the side end connecting piece 42a is connected to the first side connecting piece 46a, and a gap is reserved between the first side connecting piece 46a and a vertex 4211 of the edge wave angle 421, so that the sheath size at corners of the mesh cover 4 is not increased, a large mesh hole is formed between the side end connecting piece 42a and the adjacent hooking unit 47, and the edge wave angle 421 fixed by suturing is prevented from moving relative to a groove 5 and piercing and damaging a biological tissue when the mesh cover 4 is compressed or significantly deformed.

In this embodiment, if the maximum axial length of at least one end gap is greater than the maximum axial length of a middle gap, the maximum gap distance between the first side connecting piece 46a and the adjacent second side connecting piece 46b is also greater than the maximum gap distance between two adjacent second side connecting pieces 46b, which helps increase the size of a side mesh hole close to the axial end of the mesh cover 4 to facilitate entry of the guide wire and the bridging stent. In other embodiments, the maximum gap distance between the first side connecting piece 46a and the adjacent second side connecting piece 46b may be basically equal to the maximum gap distance between two adjacent second side connecting pieces 46b.

### Embodiment III

Referring to FIG. 4 and FIG. 6, in this embodiment, the structure of a lumen stent 100 is basically the same as those of the lumen stents in Embodiment I and Embodiment II. The difference is that in this embodiment, a trough 4711 of a first hooking piece 471 and a crest 4721 of a second hooking piece 472 both include transverse rods 473. Two first wave rods 4712 of the first hooking piece 471 are connected through the transverse rod 473, and two second wave rods 4722 of the second hooking piece 472 are connected through the transverse rod 473. By the transverse rod 473, a transitional connection distance is reserved between the two wave rods that are connected to each other, so that a reserved hooking gap also has a distance in the horizontal direction. In this way, the size of a mesh hole formed by the hooking gap is further increased, and then the mesh hole becomes more suitable as an entry point for a bridging stent. In a case that the transverse rod 473 is not arranged, the two wave rods are connected directly, the wave angle of a crest 4721 or trough 4711 formed by connection is sharp. When bent along with the lumen stent, the mesh cover 4 will upwarp on a curved surface, and the wave angle will scratch an inner wall of a blood vessel and cause unnecessary damage when the lumen stent is used for a long time.

The included angle between the transverse rod 473 and the first wave rod 4712 and/or the second wave rod 4722 is an obtuse angle, so that the size of the mesh hole formed by the hooking gap is further increased. The length of the transverse rod 473 is appropriately set, which ranges from 1 mm to 4 mm. This design can not only achieve the effect of increasing the hooking gap and protecting the blood vessel, but also avoid the problem of an excessive radial compression size of the mesh cover 4 due to the excessively long transverse rod 473.

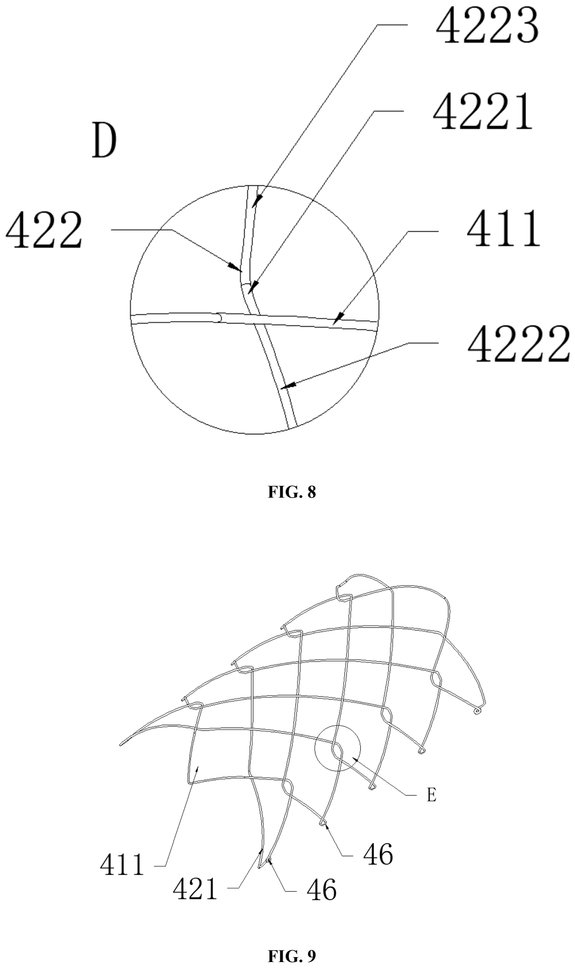

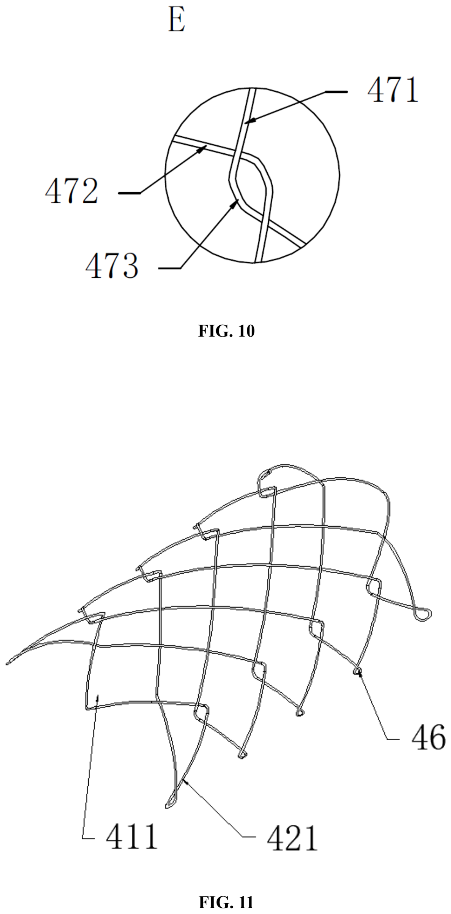

Referring to FIG. 9 and FIG. 10, preferably, the transverse rod 473 is a straight rod. The straight rod can effectively avoid scratching the blood vessel wall. In a case that the straight rod is adopted, the straight rod is connected to the two wave rods through arc transition.

Referring to FIG. 4 and FIG. 6, preferably, the transverse rod 473 is an arc-shaped rod with a low degree of bending. Preferably, the angle ranges from 60° to 140°.

### Embodiment IV

Referring to FIG. 3, FIG. 4, and FIG. 8, in this embodiment, based on any one of Embodiment I to Embodiment III, the mesh cover 4 further includes a bending portion 422, which includes a first rod 4222 and a second rod 4223 that are connected to each other. A bending angle 4221 is formed between the first rod 4222 and the second rod 4223. The first rod 4222 extends from the bending angle 4221 toward one axial end of the mesh cover 4, and the second rod 4223 extends from the bending angle 4221 toward the other axial end of the mesh cover 4. Because the first rod 4222 and the second rod 4223 of the bending portion 422 respectively extend toward two opposite axial ends of the mesh cover 4, compared with a straight rod, when the mesh cover 4 is squeezed by a blood vessel wall in a radial direction and is bent to conform with a shape of a blood vessel, the first rod 4222 and the second rod 4223 that form the bending angle 4221 can move relative to each other. In this way, the bending portion 422 can bulge away from a groove 5. The mesh cover 4 can be easily deformed to form an arch in this region, thereby avoiding compression of the space in the groove 5 and allowing a guide wire and a bridging stent to conveniently enter the groove 5. Particularly, if the bending angle 4221 formed by the bending portion 422 is an obtuse angle, the first rod 4222 and the second rod 4223 can move relative to each other in larger space, and the mesh cover can be deformed more flexibly. Furthermore, when the bending portion 422 bulges away from the groove 5, a sharp structure is not formed to damage the blood vessel wall.

In this embodiment, the bending angle 4221 of the bending portion 422 is located close to the axial end of the mesh cover 4. For example, the bending angle 4221 is located between the axial end of the mesh cover 4 and a hooking unit 47 closest to the axial end. When a branch stent 6 is arranged near the axial end of the mesh cover 4, the bending portion 422 can form an arch structure bulging away from the groove 5 after a lumen stent 100 is implanted, which provides larger space in the groove 5 for a branch port facing the groove 5 of the branch stent 6 to allow the guide wire and the bridging stent to conveniently enter the branch stent 6.

Further, the bending angle 4221 of the bending portion 422 is bent toward the axial end, close to the bending angle, of the mesh cover 4. Through such an arrangement, sizes of mesh holes near the axial end are more uniform. In other embodiments, the bending angle 4221 of the bending portion 422 is bent toward the axial end, away from the bending angle, of the mesh cover 4.

The first rod 4222 of the bending portion 422 extends from the bending angle 4221 toward one axial end of the mesh cover 4, and the second rod 4223 extends from the bending angle 4221 toward the other axial end of the mesh cover 4. Meanwhile, the first rod 4222 of the bending portion 422 extends toward one radial edge of the mesh cover 4, and the second rod 4223 extends toward the other radial edge of the mesh cover 4. Through such an arrangement, the first rod 4222 and the second rod 4223 both obliquely extend relative to an axial direction and the radial direction of the mesh cover 4, so that the bending portion 422 can better conform to radial deformation of the mesh cover 4, and can better conform to axial bending and deformation of the mesh cover 4.

An end that is away from the bending angle 4221 of the first rod 4222 of the bending portion 422 is connected to an edge of the groove 5. Through such an arrangement, the edge of the groove 5 can support the first rod 4222. When the groove 5 is subjected to a radial force, the end that is away from the bending angle 4221 of the first rod 4222 can better transfer the radial force, so that the first rod 4222 can be deformed more flexibly along with the groove 5.

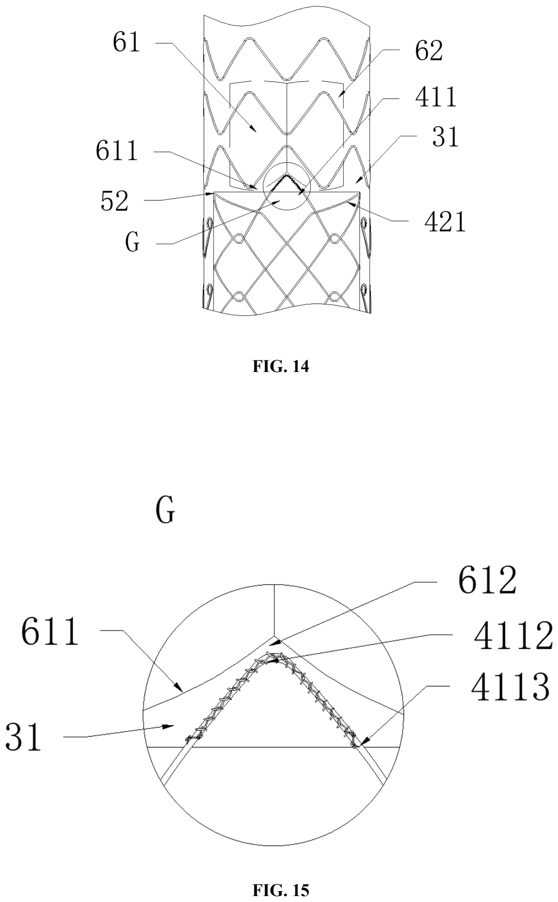

Referring to FIG. 4 and FIG. 8, exemplarily, in this embodiment, an edge wave angle 421 includes a vertex 4211 and two third wave rods 4212 connected to the vertex. The first rod 4222 serves as one third wave rod 4212 of the edge wave angle 421, and the other third wave rod 4212 of the edge wave rod 421 extends from the vertex 4211 toward the axial end away from the vertex 4211 of the mesh cover 4. The two third wave rods 4212 form an included angle at the vertex 4211 of the edge wave angle 421. The included angle formed at the vertex 4211 of the edge wave angle 421 is appropriately set. If the included angle is too large, on the one hand, the sizes of mesh holes on two sides of the first 4222 are not uniform, and on the other hand, the first rod 4222 may extend approximately in the radial direction, so that it is difficult to retract the lumen stent 100 into a sheath. If the included angle is too small, the sizes of the mesh holes on two sides of the first rod 4222 are not uniform, and the vertex 4211 of the edge wave angle 421 may easily pierce and injure a blood vessel. Therefore, the included angle may be set to an acute angle, which may range, for example, from 30° to 70°. Within this range, the sizes of the mesh holes on two sides of the first rod 4222 are uniform, and it is easy to retract the lumen stent 100 into the sheath, to increase the safety of the lumen stent. The edge wave angle 421 is connected to a corner 52 (see FIG. 14) of the groove 5. For example, the vertex 4211 of the edge wave angle 421 is connected to the corner of the groove 5 to support the corner of the groove 5. In this way, the corner of the groove 5 can be fully unfolded to better maintain the shape of an opening of the groove 5.

The second rod 4223 of the bending portion 422 passes through a first mesh region 41 and is connected to a vertex of a first hooking piece 471 or second hooking piece 472 on an opposite side. For example, a part of the second rod 4223 serves a support wire in the first mesh region 41, and a part of the second rod 4223 serves a wave rod of the hooking unit. Through such an arrangement, the vertex of the first hooking piece 471 or the second hooking piece 472 provides certain supporting for the second rod 4223. When the groove 5 and the mesh cover 4 are subjected to a radial force, an end that is away from the bending angle 4221 of the second rod 4223 can better transfer the radial force, so that the second rod 4223 can be deformed more flexibly along with the mesh cover 4. In addition, the second rod 4223 passes through the first mesh region 41 and extends to the opposite side, so that the first rod 4222 and the second rod 4223 can respectively transfer a squeezing force applied to two radial sides of the mesh cover 4, and can be deformed accordingly to better maintain the space in the groove 5.

In this embodiment, the mesh cover 4 includes four side end connecting pieces 42a and four bending portions 422. Two side end connecting pieces 42a and two bending portions 422 are located at a proximal end of the mesh cover 4, the two side end connecting pieces 42a are respectively connected to two corners at the proximal end of the groove 5, and the two bending portions 422 are respectively connected to the two side end connecting pieces 42a. Two side end connecting pieces 42a and two bending portions 422 are located at a distal end of the mesh cover 4, the two side end connecting pieces 42a are respectively connected to two corners at the distal end of the groove 5, and the two bending portions 422 are respectively connected to the two side end connecting pieces 42a. In other embodiments, the number of side end connecting pieces 42a and the number of bending portions may be selected according to an actual application scenario.

### Embodiment V

Referring to FIG. 3 and FIG. 4, in this embodiment, based on any one of Embodiment I to Embodiment IV, the mesh cover further includes a side end connecting piece 42a and a middle end connecting piece 41a that are connected to a main stent 10. The side end connecting piece 42a is closer to a radial edge of the mesh cover 4 compared with the middle end connecting piece 41a. Compared with the solution in which the middle end connecting piece 41a is not connected to the main stent, because the middle end connecting piece 41a is connected to the main stent 10, when bent, the main stent 10 can better drive the mesh cover 4 to bend and deform to conform with the shape of a blood vessel. Furthermore, the middle end connecting piece 41a connected to the main stent 10 can provide better support for an axial end of the groove 5, to avoid the formation of a gap between the main stent 10 and the middle end connecting piece 41a in an axial direction when a lumen stent 100 is bent. In this way, the possibility that an inner wall of a blood vessel with a narrow true lumen squeezes the gap and enters the groove 5 through the gap, which may prevent a guide wire and a bridging stent from entering a branch stent 6, is avoided.

Further, the middle end connecting piece 41a at a proximal end of the mesh cover 4 is closer to a proximal end of the lumen stent 100 compared with the side end connecting piece 42a at the proximal end of the mesh cover 4, and/or the middle end connecting piece 41a at a distal end of the mesh cover 4 is closer to a distal end of the lumen stent 100 compared with the side end connecting piece 42a at the distal end of the mesh cover 4. Compared with the solution in which axial ends of the side end connecting piece 42a and the middle end connecting piece 41a are flush, according to the solution of this embodiment, the mesh cover 4 has a larger axial size at a position of the middle end connecting piece 41a, so that the mesh cover 4 can form a better arch structure after being bent along with the main stent 10, an axial end of the mesh cover 4 can be effectively prevented from forming an approximate plane when the main stent 10 is bent, the internal space of the groove 5 is better maintained to allow the guide wire and the bridging stent to conveniently enter the groove. Furthermore, after being implanted, the bridging stent is prevented from being squeezed excessively.

Referring to FIG. 3, exemplarily, the middle end connecting piece 41a includes a middle wave angle 411, and the side end connecting piece 42a includes an edge wave angle 421. The vertex 4112 of the middle wave angle 411 at the proximal end of the mesh cover 4 is closer to the proximal end of the lumen stent 100 compared with the vertex 4211 of the edge wave angle 421, and the vertex 4112 of the middle wave angle 411 at the distal end of the mesh cover 4 is closer to the distal end of the lumen stent 100 compared with the vertex 4211 of the edge wave angle 421. Compared with the solution in which the vertex 4112 of the middle wave angle 411 and the vertex 4211 of the edge wave angle 421 are flush (see FIG. 2), after the mesh cover 4 is bent, the middle wave angle 411 protrudes for a longer length, thereby reducing an axial stretching degree of the edge wave angles 421 on two sides. In this way, the shapes and sizes of mesh holes in the region where the edge wave angle 421 is located can be better maintained to facilitate passage of the guide wire and the bridging wire through the mesh holes. Further, the middle wave angle 411 in the middle provides a sufficient stretching length, which can ensure that the proximal end and the distal end of the mesh cover 4 will not be stretched and deformed excessively toward an inner cavity of the groove 5, and can effectively prevent the axial end of the mesh cover 4 from forming an approximate plane when the main stent 10 is bent, but instead allow formation of a better arch structure. In this way, the internal space formed by the proximal end and the distal end of the mesh cover 4 and the groove 5 is effectively maintained.

In other embodiments, as shown in FIG. 2, the vertex 4112 of the middle wave angle 411 is flush with the vertex 4211 of the edge wave angle 421; or the vertex 4211 of the edge wave angle 421 is closer to a corresponding axial end of the lumen stent 100 compared with the vertex 4112 of the middle wave angle 411.

In an embodiment, specifically, as shown in FIG. 8, FIG. 11, and FIG. 14, the middle wave angle 411 includes two fourth wave rods 4113 connected to the vertex 4112, the vertex 4112 of the middle wave angle 411 is connected to one axial end of the groove 5, and the two fourth wave rods 4113 extend from the vertex 4112 of the middle wave angle 411 toward the other axial end of the groove 5. Further, the vertex 4112 of the middle wave angle 411 is opposite to the first mesh region 41 in the axial direction, and the two fourth wave rods 4113 extend from the vertex 4112 of the middle wave angle 411 in directions away from each other and are respectively connected to second mesh regions 42 on two radial sides of the first mesh region 41, for example, are respectively connected to hooking units 47 on two radial sides of the mesh cover 4. When the main stent 10 is bent and protrudes to a direction toward an opening of the groove 5, the middle wave angle 411 can drive the second mesh regions 42 connected to the middle wave angle 411 to bend, and simultaneously drive the first mesh region 41 connected to the second mesh regions 42 to bend. Because each second mesh region 42 includes a plurality of hooking units 47, when the middle wave angle 411 is connected to the hooking unit 47, the middle wave angle can drive mesh holes in the second mesh region 42 to fully expand when bent to facilitate passage of the guide wire and the bridging stent through the mesh holes. Meanwhile, the hooking unit 47 can limit the stretching length of the second mesh region 42 and further limit the stretching length of the first mesh region 41 connected to the second mesh region, thereby avoiding the problems of compression of the internal space of the groove 5 due to excessive stretching of the first mesh region 41 and excessive small retraction of the mesh holes in the first mesh region 41. In this way, the internal space of the groove 5 and the shapes of the mesh holes in the first mesh region 41 can be better maintained to further facilitate passage of the guide wire and the bridging stent through the mesh holes.

As shown in FIG. 3, FIG. 4, and FIG. 6, two fourth wave rods 4113 of the middle wave angle 411 at the proximal end of the mesh cover 4 extend from the vertex 4112 of the middle wave angle 411 in directions away from each other to form first wave rods 4712 of two hooking units 47 on two radial sides of the mesh cover 4. Two fourth wave rods 4113 of the middle wave angle 411 at the distal end of the mesh cover 4 extend from the vertex 4112 of the middle wave angle 411 in direction away from each other to form second wave rods 4722 of the two hooking units 47 on two radial sides of the mesh cover 4. The two fourth wave rods 4113 of the middle wave angle 411 may be respectively substantially parallel to a first directional support wire 481 and a second directional support wire 482 in the first mesh region 41. In other embodiments, the two fourth wave rods 4113 of the middle wave angle 411 may be not parallel to the first directional support wire and the second directional support wire in the first mesh region 41. The angle (namely, the included angle formed by the two fourth wave rods 4113 at the vertex 4112 of the middle wave angle 411) of the middle wave angle 411 is appropriately set. If the angle of the middle wave angle 411 is too large and the middle wave angle is connected to a main membrane 31 by suturing, the middle wave angle 411 is likely to slide relative to the main membrane 31 in a length direction of the fourth wave rod 4113. If the angle of the middle angle 411 is too small, the middle angle 411 is likely to pierce the main membrane 31 and damage a blood vessel wall. Therefore, the angle of the middle wave angle 411 may range from 20° to 80°. On the one hand, the middle wave angle 411 is stably fixed, and on the other hand, the middle wave angle 411 is prevented from piercing the main membrane 31 and damaging the blood vessel.

Referring to FIG. 8, further, in this embodiment, the mesh cover 4 includes the bending portion 422 of Embodiment IV, and the two fourth wave rods 4113 of the middle wave angle 411 respectively span over the upper side of the bending portion 422. In this embodiment, the two fourth wave rods 4113 of the middle wave angle 411 respectively span over the upper side of second rods 4223 of two bending portions 422. In other embodiments, the two fourth wave rods 4113 of the middle wave angle may span over the upper side of first rods 4222 of the two bending portions 422. As long as the two fourth wave rods 4113 of the middle wave angle 411 respectively span over the upper side of the bending portion 422, through such arrangement, when the mesh cover 4 is compressed or bent in the radial direction, the bending portion 422 can bulge away from the groove 5, to lift the fourth wave rods 4113 that span over the upper side of the bending portion, thereby preventing the middle wave angle 411 from being excessively stretched after the lumen stent 100 is bent, which may otherwise form a relatively flat structure. Therefore, the mesh cover 4 can better maintain the internal space of the groove 5 at the axial ends, and avoid occupation of space near a port of the branch stent 6.

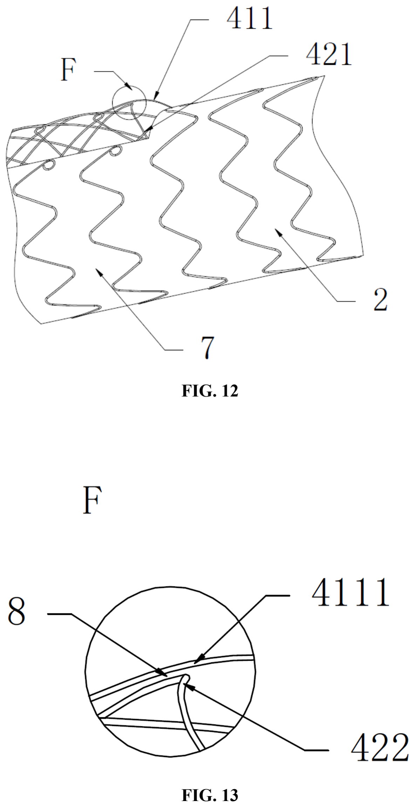

When the lumen stent 100 is in an unfolded state, the fourth wave rod 4113 of the middle wave angle 411 may be in contact with the bending portion 422. Alternatively, as shown in FIG. 12 and FIG. 13, a gap 8 is reserved between the fourth wave rod 4113 of the middle wave angle 411 and the bending portion 422 in the radial direction of the lumen stent 100. Regardless of whether they are in contact with each other or form the gap 8, the middle wave angle 411 can move relative to the bending portion 422. In this way, the mesh cover 4 has better deformation capability and compliance both in a bent state and a straight state, and can better maintain the internal space formed between the mesh cover 4 and the groove 5. If the gap 8 is reserved between the fourth wave rod 4113 of the middle wave angle 411 and the bending portion 422 in the radial direction of the lumen stent 100, the reserved gap 8 allows the bending portion 422 to have larger movement space, so that when bent away from the groove 5, the mesh cover 4 can better form an arch structure to allow the guide wire and the bridging stent to enter the branch stent 6 conveniently.

Referring to FIG. 12 and FIG. 13, in an embodiment, the fourth wave rod 4113 of the middle wave angle 411 may be an arc-shaped rod 4111, which is an arch curved structure protruding upward, specifically, protruding away from the center axis of the lumen stent 100 in the radial direction of the lumen stent 100. The protruding arch curved structure is more conducive to formation of the gap 8 between the middle wave angle 411 and the bending portion 422. Further, the arc-shaped rod 4111 of the arch curved structure enables the axial end of the mesh cover 4 to form an arch structure to conform with the arc-shaped rod 4111 after the mesh cover 4 is bent in the axial direction along with bending of the main stent 10, to provide stronger support. In this way, the internal space at the axial end of the groove 5 can be better maintained to prevent the internal space from being squeezed excessively.

Referring to FIG. 14 and FIG. 15, the branch stent 6 at a proximal section 2 of the main stent 10 is a stent with two branch ports 61. The stent with two branch ports 61 is formed by connecting two stents with a single branch port 62 side by side to an inner wall of the proximal section 2 of the lumen stent 100 by suturing or bonding. Branch ports of the two stents with a single branch port 62 are usually set to be approximately circular. A gap position 612 is reserved between the two branch ports. When the stent with two branch ports 61 is sutured to a proximal main membrane of the lumen stent 100 at a proximal edge of the groove 5, the gap position 612 forms a blank section of the proximal main membrane. When the mesh cover 4 is installed in the groove 5, the middle end connecting piece 41a of the mesh cover 4 is connected to the proximal main membrane at the gap position 612. For example, the vertex 4112 of the middle wave angle 411 extends to the gap position 612, so that the vertex 4112 of the middle wave angle 411 is closer to the proximal end of the lumen sent 100 compared with the proximal edge of the groove 5, and the middle wave angle 411 is connected to the proximal main membrane at the gap position 612. Through such an arrangement, the branch port 611 of the stent with two branch ports 61 can support the middle wave angle 411, and can prevent the middle wave angle 411 from piercing the main membrane 31.

It may be understood that the foregoing middle end connecting piece 41a may be connected to an inner wall or outer wall of the main membrane 31. For example, the vertex 4112 of the middle wave angle 411 and a part of the fourth wave rod 4113 extend to the inner wall of the main membrane 31 at the gap position 612, that is, the main membrane 31 at the gap position 612 covers outer sides of the vertex 4112 of the middle wave angle 411 and the part of the fourth wave rod 4113, and the vertex 4112 of the middle wave angle 411 and the part of the fourth wave rod 4113 are connected to the inner wall of the main membrane 31 by suturing. Alternatively, the main membrane 31 at the gap position 612 covers inner sides of the vertex 4112 of the middle wave angle 411 and the part of the fourth wave rod 4113, and the vertex 4112 of the middle wave angle 411 and the part of the fourth wave rod 4113 are connected to the outer wall of the main membrane 31 by suturing. If the middle end connecting piece 41a is connected to the inner wall of the main membrane 31, the middle end connecting piece 41a will not warp outward nor damage an inner wall of a blood vessel when the lumen stent 100 is deformed, which improves the safety of the lumen stent.

In another embodiment, the branch stent 6 at the proximal section 2 of the main stent 10 may be not the stent with two branch ports 61, and the branch stent 6 at a distal section 1 is the stent with two branch ports 61. In this way, the middle wave angle 411 at the distal end of the mesh cover 4 may be connected to the main stent 10 by the foregoing connection method. In other embodiments, the connection position and the connection method of the middle wave angle 411 and the main stent 10 are not limited thereto, and may be appropriately selected as needed.

Further, a support piece is arranged at an edge of the branch port 611 of the stent with two branch ports 61, which can better maintain the shape of the branch port 611. At least a partial region of the support piece is connected to the main membrane 31 and is located on two sides of the middle end connecting piece 41a, to better support the middle wave angle 411.

The foregoing specific embodiments are merely some embodiments of the present disclosure and are not intended to limit the present disclosure. The description cannot be an exhaustive list of all embodiments of the present disclosure. Some features of the foregoing different embodiments may be replaced or combined with each other. Those skilled in the art may also make simple replacements according to actual needs. The concept of the present disclosure shall be subject to the claimed scope of protection.

## Claims

1. A lumen stent, **characterized by** comprising a mesh cover, wherein the mesh cover comprises at least one column of hooking units, each hooking unit comprises a first hooking piece and a second hooking piece that are hooked to each other in sequence from a proximal end to a distal end, the first hooking piece comprises one trough and two first wave rods connected to the trough, the second hooking piece comprises one crest and two second wave rods connected to the crest, and the trough of the first hooking piece and the crest of the second hooking piece are configured to move relative to each other in an axial direction; and the mesh cover comprises a proximal cover and a distal cover in the axial direction, in the proximal cover, a first wave rod that is closer to a radial edge of the mesh cover in a hooking unit spans over an upper side of a second wave rod in the same hooking unit; and/or in the distal cover, a second wave rod that is closer to the radial edge of the mesh cover in a hooking unit spans over an upper side of a first wave rod in the same hooking unit.

2. The lumen stent according to claim 1, **characterized in that** a hooking gap is reserved between the first hooking piece and the second hooking piece in the same hooking unit.

3. The lumen stent according to claim 1, **characterized in that** the trough and the crest comprise transverse rods, the two first wave rods are connected through the transverse rod, and/or the two second wave rods are connected through the transverse rod.

4. The lumen stent according to claim 3, **characterized in that** included angles between the first wave rods and the transverse rod are obtuse angles, and/or included angles between the second wave rods and the transverse rod are obtuse angles.

5. The lumen stent according to claim 3, **characterized in that** the transverse rod is a straight rod or an arc-shaped rod.

6. The lumen stent according to claim 1, **characterized in that** the mesh cover comprises a first mesh region and at least two second mesh regions respectively connected to two sides of the first mesh region in a circumferential direction of the lumen stent; and each second mesh region comprises at least one column of hooking units, and the first mesh region comprises a plurality of columns of cross units.

7. The lumen stent according to claim 1, **characterized in that** an axial gap is reserved between the first wave rod and the second wave rod close to the radial edge of the mesh cover of the hooking unit, a plurality of axial gaps formed by the same column of hooking units comprise end gaps and a middle gap, the end gaps are closer to an axial end of the mesh cover compared with the middle gap, and the maximum axial length of at least one end gap is greater than the maximum axial length of the middle gap.

8. The lumen stent according to claim 1, **characterized in that** the mesh cover further comprises at least one column of side connecting pieces arranged on a radial side of the mesh cover, and the side connecting pieces are connected to the hooking units and comprise connecting holes for connection.

9. The lumen stent according to claim 8, **characterized in that** the mesh cover further comprises a side end connecting piece arranged at an axial end of the mesh cover, a plurality of side connecting pieces in the same column comprise two first side connecting pieces and a second side connecting piece between the two first side connecting pieces, each first side connecting piece is connected to the side end connecting piece and the hooking unit, respectively, in the axial direction, and the second side connecting piece is connected to one first hooking piece and one second hooking piece, respectively.

10. The lumen stent according to any one of claims 1 to 9, **characterized in that** further comprising a main stent, which comprises an inner cavity, wherein side surfaces of the main stent sink inward in a radial direction to form a groove, the mesh cover is connected to the groove, and a radial gap is reserved between at least part of the mesh cover and the bottom of the groove in the radial direction of the lumen stent and is communicated with the inner cavity.

11. The lumen stent according to claim 10, **characterized in that** the mesh cover is connected to the main stent through the plurality of side connecting pieces, and a gap reserved between two adjacent side connecting pieces is communicated with the radial gap.
